# EUROPÄISCHE PATENTSCHRIFT

(11) **EP 2 164 386 B1**
(45) Veröffentlichungstag und Bekanntmachung des Hinweises auf die Patenterteilung: **27.04.2011**
(21) Anmeldenummer: 08773463.8
(22) Anmeldetag: 16.06.2008
(51) Int. Cl.: A61B 5/00, A61B 5/11, A61B 7/00

(54) **VORRICHTUNG UND VERFAHREN ZUR VORHERSAGE EINES KONTROLLVERLUSTES ÜBER EINEN MUSKEL**
APPLIANCE AND METHOD FOR PREDICTING A LOSS OF CONTROL OVER A MUSCLE
DISPOSITIF ET PROCÉDÉ DE PRÉDICTION D'UNE PERTE DE CONTRÔLE SUR UN MUSCLE

(30) Priorität: 11.07.2007 DE 102007032268; 14.08.2007 DE 102007038392
(43) Veröffentlichungstag der Anmeldung: 24.03.2010
(73) Patentinhaber: Fraunhofer-Gesellschaft zur Förderung der angewandten Forschung e.V., 80686 München (DE); Friedrich-Alexander-Universität Erlangen-Nürnberg, 91054 Erlangen (DE)
(72) Erfinder: MÖRSDORF, Hans-Joachim, 90763 Fürth (DE); ASCHENBRENNER, Stefan, 90542 Eckental (DE); THIELECKE, Jörn, 91056 Erlangen (DE); SCHMITT, Huber, 91056 Erlagen (DE)
(74) Vertreter: Zimmermann, Tankred Klaus
(86) Internationale Anmeldenummer: PCT/EP2008/004832
(87) Internationale Veröffentlichungsnummer: WO 2009/006980

(56) Entgegenhaltungen:
- EP-A- 1 408 443
- DE-A1-102006 002 114
- JP-A- 2004 364 009
- JP-A- 2007 142 540
- JP-A- 2007 167 185
- US-A- 4 748 987
- US-A- 4 805 636
- US-A1- 2004 127 807
- US-B1- 6 477 406
- US-B1- 7 177 686
- AXISA F ET AL: "Flexible Technologies and Smart Clothing for Citizen Medicine, Home Healthcare, and Disease Prevention" IEEE TRANSACTIONS ON INFORMATION TECHNOLOGY IN BIOMEDICINE, IEEE SERVICE CENTER, LOS ALAMITOS, CA, US, Bd. 9, Nr. 3, 1. September 2005 (2005-09-01), Seiten 325-336, XP011138579 ISSN: 1089-7771
- PASCAL MADELEINE ET AL: "Spectral moments of mechanomyographic signals recorded with accelerometer and microphone during sustained fatiguing contractions" MEDICAL & BIOLOGICAL ENGINEERING & COMPUTING, SPRINGER, BERLIN, DE, Bd. 44, Nr. 4, 1. April 2006 (2006-04-01), Seiten 290-297, XP019360730 ISSN: 1741-0444
- SILVA J ET AL: "Coupled microphone-accelerometer sensor pair for dynamic noise reduction in MMG signal recording" ELECTRONICS LETTERS, IEE STEVENAGE, GB, Bd. 39, Nr. 21, 16. Oktober 2003 (2003-10-16), Seiten 1496-1498, XP006021196 ISSN: 0013-5194

## Beschreibung

Die vorliegende Erfindung bezieht sich auf eine Vorrichtung und auf ein Verfahren zur Vorhersage eines Verlustes einer Kontrolle über eine Muskulatur und insbesondere auf eine Detektion von Schwindelanfällen und Müdigkeit. Schwindelanfälle können beispielsweise im Vorfeld eines Sturzes auftreten, währenddessen Ermüdungszustände von Muskeln z.B. als Folge von körperlichen Anstrengungen und Müdigkeit - wie beispielsweise während des Autofahrens - auftreten können. Weitere Anwendungsfelder umfassen beispielsweise ein Erkennen eines Kontrollverlustes unter Einfluss von Drogen oder Alkohol und ggf. ein Erkennen degenerativer neurologischer Erkrankungen wie beispielsweise Parkinson.

Viele ältere Menschen hegen den Wunsch, so lange wie möglich in ihrer Wohnung bleiben zu können. Dabei stellt sich jedoch für die Angehörigen und das medizinische Personal die Frage, wie beispielsweise ein spontan geänderter Gesundheitszustand erkannt werden kann. Häufig ergibt sich ein Problem dadurch, dass, wenn beispielsweise Senioren stürzen, sie hilflos am Boden liegen bleiben können. Zahlreiche Anstrengungen wurden unternommen, um diese Situation beispielsweise mittels eines Sturzdetektors zu erkennen, um somit automatisch Hilfe anzufordern zu können. Diese Anstrengungen umfassen beispielsweise eine Sturzdetektion mittels Beschleunigungs- und/oder Lagesensoren. Dabei wird bei Überschreiten eines bestimmten Grenzwertes, wie beispielsweise eines Aufpralls auf dem Boden, ein Alarm ausgelöst. Weitere konventionelle Verfahren versuchen zusätzlich anhand von Lagesensoren die Raumlage des Menschen bzw. eventuelle Bewegungsmuster im Anschluss an ein vorausgegangenes Sturzereignis auszuwerten, um dadurch eine höhere Zuverlässigkeit zu erreichen und Fehlalarme auszuschließen. Es zeigt sich jedoch, dass sich mittels konventioneller Vorrichtungen oder Verfahren ein Sturz bzw. ein Torkeln nur im Nachhinein feststellen lässt und sich dadurch der Sturz selbst kaum vermeiden lässt. Besser wäre eine Vorrichtung, die in der Lage ist, einen Sturz bzw. ein Torkeln "vorauszuahnen", um damit den Sturz selbst verhindern zu können. Eine derartige Vorhersageeinrichtung, könnte neben den adressierten Krankheitsbildern auch dahin gehend angewendet werden, um beispielsweise einem in Folge einer Übermüdung auftretenden Kontrollverlust vorzubeugen.

Ausgehend von diesem Stand der Technik liegt der vorliegenden Erfindung die Aufgabe zugrunde, eine Vorrichtung und ein Verfahren zu schaffen, die einen Verlust der Kontrolle über eine Muskulatur vorherzusagen kann und die gleichzeitig an einen menschlichen Körper leicht anbringbar und einfach handhabbar ist.

Diese Aufgabe wird durch eine Vorrichtung nach Anspruch 1, ein Verfahren nach Anspruch 24 oder ein Computerprogramm nach Anspruch 28 gelöst.

Der vorliegenden Erfindung liegt die Erkenntnis zugrunde, dass ein bevorstehender Verlust einer Kontrolle über einen Muskel eines Menschen oder eines menschlichen Körpers sich durch eine Änderung der Geräusche bzw. der Geräuschmuster in der Muskulatur ankündigt, die wiederum mit Torkelbewegungen einhergehen können. Somit ist ein Kontrollverlust eines Muskels (z.B. Stützmuskel oder Beinmuskulatur) dadurch vorhersagbar, dass eine Einrichtung Geräusche des Muskels detektiert und eine weitere Einrichtung eine Beschleunigung z.B. des menschlichen Körpers oder des Kopfes erfasst. Die detektierten Daten (Muskelgeräusche und Beschleunigung) können in einer Auswerteeinheit derart ausgewertet werden, dass ein bevorstehender Kontrollverlust über die Muskulatur aus den (typischen) Geräuschen der Muskulatur und der Beschleunigung vorhergesagt werden kann.

Somit umfassen Ausführungsbeispiele der vorliegenden Erfindung eine Datenfusion von Muskelgeräuschen und Beschleunigungsmessdaten, so dass es ermöglicht wird, einen beobachteten Menschen prinzipiell bereits vor dem Sturz zu warnen.

Eine Messwertaufnahme kann beispielsweise auf zweierlei Art geschehen:
(a) Einzelsensoren zur Erfassung von Muskelgeräuschen und Körperbeschleunigung sind getrennt am menschlichen Körper angebracht;
(b) ein kombinierter Sensor, der den Geräuschsensor und den Beschleunigungssensor umfasst, wird an einer Stelle des menschlichen Körpers angebracht.

Im Fall (b), in dem die Sensoren als Kombisensor ausgeführt sind, erscheint eine Fixierung am Oberschenkel des Menschen sinnvoll. Hier ist sowohl eine Detektion von Bewegungsmustern als auch die Detektion eines Tonusverlustes, der ein mögliches Torkeln verursachen kann, möglich. Eine Qualität der Regelung des Muskeltonus (Muskelspannung) bestimmt sich aus der Einhaltung eines Arbeitspunktes, der durch ein Gleichgewicht zwischen der Schwerkraftkomponente und der Kraftwirkung infolge der Muskelspannung bestimmt ist. Bei einem Tonus hoher Qualität ist die Muskelspannung hoch genug, um der Schwerkraft zu widerstehen, andererseits jedoch auch niedrig genug sein, um eine harmonische Bewegung zu ermöglichen. Ein Krampf ist beispielsweise eine starke überhöhung des Tonus und eine Paralyse stellt beispielsweise einen Verlust des Tonus dar.

Bewegungsmuster können beispielsweise auf ein Stehen, Gehen oder Laufen oder auch Treppensteigen hinweisen und können dementsprechend in der Auswerteeinheit entsprechend detektiert werden. Neben der Fixierung des kombinierten Sensors an dem Oberschenkel des Menschen können optional auch andere Stellen am menschlichen Körper zur Platzierung des kombinierten Sensors benutzt werden - beispielsweise am Torso oder im Hals/Schulter-Bereich. Zum gegenwärtigen Zeitpunkt erscheint jedoch eine Fixierung am Oberschenkel des Menschen als sinnvoll.

Im Fall (a), bei dem die Sensoren (Beschleunigungssensor, Geräuschsensor) als Einzelsensoren ausgeführt sind, liegt der Ansatz nahe, ein Mikrofon (als Geräuschsensor) zur Aufzeichnung der Muskelgeräusche auch in diesem Fall am Oberschenkel zu platzieren und den Beschleunigungssensor an der Hüfte des Menschen anzubringen. Somit können Bewegungen des Torsos (beispielsweise in Folge eines Sturzes, Drehung, etc) klassifiziert werden. Aber auch hier können bei weiteren Ausführungsbeispielen die Einzelsensoren an anderen Stellen platziert werden, die beispielsweise entsprechend einem konkreten Anwendungsfall gewählt sind.

Neben der bereits erwähnten Anwendungsmöglichkeit als eine Vorhersageeinrichtung, die vor einem bevorstehenden Sturz warnen kann, können Ausführungsbeispiele beispielsweise herzbedingte Schwindelanfälle feststellen. Dies kann beispielsweise durch einen weiteren Sensor zur Detektion von Herzmuskelgeräuschen geschehen, der die im Alter häufig auftretenden herzbedingten Schwindelanfälle bereits vor Eintritt eines manifesten Schwindelanfalls registriert und durch ein präventives Verfahren eingreifen kann. Das präventives Verfahren kann beispielsweise ein entsprechendes Warnsignal umfassen, so dass im Vorfeld des Schwindelanfalls Vorkehrungen getroffen werden können bzw. eine Warnung erfolgen kann.

Neben diesen alters- bzw. krankheitsbedingten Symptomen, sind Ausführungsbeispiele der vorliegenden Erfindung ebenfalls geeignet, eine Müdigkeit beispielsweise beim Autofahren zu erkennen und anhand von Nickbewegungen des Kopfes zu detektieren. Für dieses Anwendungsszenario können beispielsweise der Geräuschsensor bzw. Beschleunigungssensor am Kopf bzw, im Hals-Schulter-Bereich angeordnet werden. Neben Ermüdungserscheinungen beim Führen eines Fahrzeuges können aber ebenso muskulöse Erschöpfungszustände - beispielsweise nach körperlicher Anstrengung (z.B. Treppensteigen) - bereits im Vorfeld detektiert werden und eine entsprechende Warnung erfolgen. Je nachdem, welche Körperregion von der körperlichen Anstrengung betroffen ist, können die Sensoren entsprechend positioniert sein.

Ausführungsbeispiele versuchen somit eine kritische Situation vorauszuahnen und bereits auftretender Schwindel, Erschöpfungszustände, Müdigkeit, etc. und die damit verbundenen kurzzeitige Torkelbewegung aufgrund eines Tonusverlustes der Haltungsmuskulatur zu erkennen.

Neben den Sensoren weisen Ausführungsbeispiele ebenfalls eine Auswerteeinheit auf, die beispielsweise in ein Gehäuse des Kombisensors oder in einem Gehäuse von einem der Einzelsensoren (des Bewegungssensors oder des Geräuschsensors) integriert sein kann. Die Auswerteeinheit erfasst dabei die detektierten Daten der Sensoren und kann sich ferner einer "Intelligenz" zur Feststellung von Schwindel bzw. eines Sturzes bedienen. Die Intelligenz kann beispielsweise mittels eines Mikrocontrollers oder eines programmierbaren Chips realisiert werden und analysiert dabei entsprechende Muster der Geräusche bzw. der Beschleunigungsdaten, die einen bevorstehenden Sturz als wahrscheinlich vorhersagen können.

Darüber hinaus kann die Auswerteeinheit Module zur drahtlosen Auslösung von Alarmen, beispielsweise ein, an eine externe Einheit übermitteltes Alarmsignal, aufweisen. Dies kann beispielsweise mittels Bluetooth geschehen. Die externe Einheit kann z.B. das Alarmsignal optisch oder akustisch darstellen, eine Benachrichtigung an eine Benachrichtigungsstelle (beispielsweise Verwandte oder eine Notfallstelle) übermitteln und/oder aber auch die Alarmsituation aufzeichnen. Ferner kann bei Ausführungsbeispielen die Auswerteeinheit die erfassten Daten abspeichern bzw. ebenfalls über eine drahtlose Verbindung an ein entsprechendes Speichermedium weiterzuleiten, so dass Tonusverluste über einen gewissen Zeitraum erfasst werden können und somit Hinweise auf ein Vorliegen einer Krankheit liefern können. Eine genaue Lokalisierung des Kombisensors bzw. der Sensoren am Körper ist wie gesagt für konkrete Anwendungsfälle durch entsprechende Studien zu bestimmen und kann beliebig dem jeweiligen Anwendungsfall angepasst werden. Die hier beschriebenen Lokalisierungen der Sensoren bzw. des Kombisensors sind dementsprechend nur Beispiele und können hinsichtlich der Wirksamkeit der Vorhersage entsprechend optimiert werden.

Eine Messwertauswertung kann wie folgt geschehen. Beide Sensoren liefern ihre jeweiligen Messwerte (Geräusche oder Geräuschmuster, Beschleunigungswerte) an den entsprechend programmierten Mikrocontroller ("Intelligenz"), der eine Fusion der Daten auf der Grundlage eines physiologischphysikalischen Modells durchführt. Ziel dieser Fusion ist es dabei, abweichende Körperbewegung im Sinne eines kurzen Kontrollverlustes der Raumlage festzustellen und somit die Erkennung von Schwindel oder von erwarteten Bewegungsmustern zu ermöglichen. Dabei kann der Mikrocontroller z.B. über einen bestimmten Zeitraum "normale" Körperbewegungen, die von Mensch zu Mensch variieren, aufzeichnen, so dass durch einen Vergleich "abnorme" Körperbewegungen, die mit einem Tonusverlust einhergehen können, festgestellt werden können. Da der Muskeltonus sich beispielsweise auch beim Hinsetzen ändert, ist gerade die Kombination mit dem Beschleunigungssensor wichtig, um nur bestimmte Ereignisse (wie beispielsweise Schwindel) festzustellen, nicht jedoch ganz natürliche Verhaltensweisen, die keinerlei krankhaften Hintergrund haben bzw. nicht auf eine Müdigkeit zurückgeführt werden können.

Die Erfassung des Muskeltonus kann wie gesagt beispielsweise durch eine Auswertung der damit assoziierten Muskelgeräusche und deren Geräuschmuster geschehen. Diese Muster oder Geräuschmuster reflektieren den jeweiligen Aktivierungsgrad des Muskels. Zur einwandfreien Fokussierung auf die Muskel- bzw. Herzgeräusche kann dabei parallel eine Messung von Umgebungsgeräuschen erfolgen. Die Umgebungsgeräusche umfassen dabei beispielsweise jene Geräusche, deren Ursprung nicht in dem menschlichen Körper oder nicht im Muskel liegt. Nach einer Berücksichtigung der übertragungscharakteristik (anderes Mikrofon, Dämpfung von Kleidung, usw.) können diese dann von dem erfassten Signal der Körpergeräusche subtrahiert werden. Diese so erhaltene differentielle Messung der Geräusche erhöht deutlich die Sensitivität der Vorrichtung - bedarf aber eines weiteren Mikrofons (an einer anderen Stelle) zur Erfassung der Umgebungsgeräusche. Alternativ können unter Ausnutzung algorithmischer Verfahren auch störende Umgebungsgeräusche herausgefiltert werden.

Die Auswertung der Daten des Beschleunigungssensors führt zu Informationen über einen Verlauf der Körperlage relativ zu dem Gravitationsfeld. Damit ist gemeint, dass die Beschleunigung, die auf den Beschleunigungssensor wirkt, eine Überlagerung der Gravitationsbeschleunigung und der relativen Beschleunigung (zum Gravitationsfeld) des menschlichen Körpers bzw. des Beschleunigungssensors darstellt. Im Falle eines Sturzes verringert sich zunächst die auf den Beschleunigungssensor wirkende Beschleunigung (wegen der Fallbeschleunigung des menschlichen Körpers), um daran anschließend zum Zeitpunkt des Aufpralls auf den Boden, deutlich erhöht zu werden. Ein deutlicher Ausschlag der Beschleunigung kann somit ein Indiz für das Vorhandensein eines Sturzes sein. Spontane Änderungen können demnach wie gerade beschrieben, erkannt werden und deren Muster kann einen Rückschluss auf Schwindel erlauben.

Hinsichtlich der Fixierung der Sensoren bzw. des Kombisensors an einem Patienten ist eine praktische Durchführung derart zu wählen, dass einerseits den Forderungen nach Ergonomie und andererseits der Notwendigkeit einer zuverlässigen Messwerterfassung Rechnung getragen wird. Dementsprechend kann in Abhängigkeit von dem jeweiligen Anwendungsfall der Ort der Fixierung der Sensoren oder des Kombisensors unterschiedlich gewählt sein. Abgesehen von der bereits beschriebenen optimierten Fixierung in Bezug auf die gewünschte Anwendung (Erfassung von Schwindelgefühlen, Erfassung von Müdigkeit, Erfassung von Herzgeräuschen bzw. Herzschwächen, etc.) ist bei der Fixierung der Sensoren ebenfalls zu berücksichtigen, dass der Patient hinsichtlich seiner Bewegungsfreiheit nicht in unzumutbarer Art und Weise eingeschränkt wird und darüber hinaus, dass die natürliche Bewegung des Patienten die Fixierung der Sensoren nicht nachteilig beeinflusst (fester Halt auch bei natürlichen Bewegungen).

Im Vergleich zu konventionellen Verfahren erhöht die Auswertung zweier nur indirekt abhängiger Modalitäten die Zuverlässigkeit des Systems und bietet wie gesagt die Möglichkeit zur Prädiktion eines eventuell bevorstehenden Sturzes oder anderer Ereignisse, die mit einem momentanen Tonusverlust einhergehen. Beispiele hierfür umfassen akute Ermüdungserscheinungen, Klärung von unklaren Symptomen kardiologischen Ursprungs, Detektion motorischer Erkrankungen oder Erschöpfungszustände.

Ausführungsbeispiele der vorliegenden Erfindung sind somit vorteilhaft gegenüber dem Stand der Technik, weil die Beschleunigungserfassung durch eine Geräuscherfassung der Muskulatur oder eines bestimmten Muskels kombiniert wird. Somit ist auf intelligente Art und Weise die einseitige Beschleunigungserfassung des Standes der Technik durch eine Geräuscherfassung kombiniert. Da eine bevorstehende Tonusschwächung des Muskels sich bereits in einem Geräuschmuster der Muskulatur zeigt, ist es somit insbesondere möglich, eine Warnung im Vorfeld zu erhalten, was konventionelle Verfahren oder Methoden nicht bieten können. Damit ist eine erfindungsgemäße Vorrichtung nicht nur für die Sturz- bzw. Schwindelvorhersage sinnvoll einsetzbar, sondern ebenso bei einer rechtzeitigen Warnung im Falle von Ermüdungserscheinungen, die insbesondere für Fahrzeugführer eine besondere Gefahrenquelle darstellen. Durch eine permanente Aufzeichnung der Messdaten können weiterhin über einen Tag verteilte Tonusverluste einer bestimmten Muskulatur untersucht und aufgezeichnet werden, was weiterhin Aufschluss für bestimmte Krankheiten liefern kann.

Ausführungsbeispiele der vorliegenden Erfindung werden nachfolgend Bezug nehmend auf die beiliegenden Zeichnungen erläutert. Es zeigen:
- Fig. 1: eine schematische Darstellung einer Erfassung von Messwerten an einem Menschen und deren Auswer- tung;
- Fig. 2A: eine mögliche Verteilung von Sensoren an ver- schiedenen Stellen des menschlichen Körpers;
- Fig. 2B: mögliche Positionen eines Kombisensors;
- Fig. 2C: mögliche Positionen eines Beschleunigungssensors;
- Fig. 2D: mögliche Positionen eines Geräuschsensors;
- Fig. 3: eine mögliche kombinierte Datenerfassung; und
- Fig. 4A-C: Ausführungsbeispiele für eine Auswertung von Beschleunigungs- und Geräuschdaten.

Bezüglich der nachfolgenden Beschreibung sollte beachtet werden, dass bei den unterschiedlichen Ausführungsbeispielen gleiche oder gleichwirkende Funktionselemente gleiche Bezugszeichen aufweisen und somit die Beschreibung dieser Funktionselemente in den verschiedenen, in den nachfolgend dargestellten Ausführungsbeispielen, untereinander austauschbar sind.

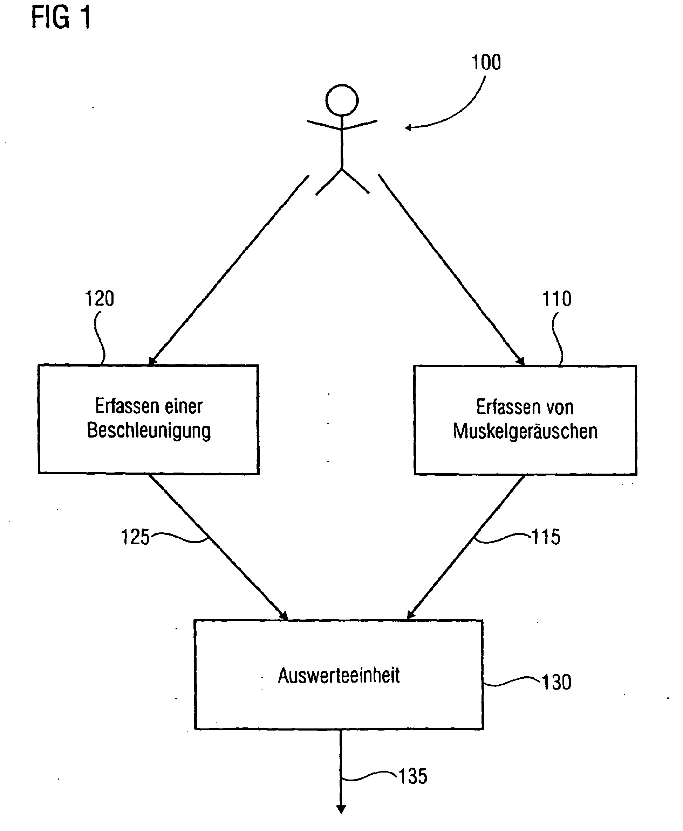

Fig. 1 zeigt eine Messwerterfassung an einem Menschen 100 oder an einem anderen lebenden Körper, welche zum einen ein Erfassen von Muskelgeräuschen durch einen Geräuschsensor 110 umfasst und zum anderen eine Erfassung einer Beschleunigung durch einen Beschleunigungssensor 120 umfasst. Der Geräuschsensor 110 kann beispielsweise im einfachsten Fall ein Mikrofon aufweisen, welches Signale 115 an eine Auswerteeinheit 130 übermittelt. Der Beschleunigungssensor 120 erfasst eine relative Beschleunigung zur Gravitationsbeschleunigung sowie körpereigene Beschleunigung, die aufgrund der Tonusvariation entsteht und übermittelt entsprechende Beschleunigungsdaten 125 ebenfalls an die Auswerteeinheit 130. Durch eine Kombination beider Daten und deren zeitlichen Verlauf (Muster) ermittelt die Auswerteeinheit 130 Ereignisdaten (Warnsignale) 135, wobei die Ereignisdaten 135 beispielsweise ein Torkeln, Schwindelgefühle, Schwindelanfälle, Ermüdungserscheinungen, etc. signalisieren können. Bei weiteren Ausführungsbeispielen sind weitere Beschleunigungssensoren und/oder weitere Geräuschsensoren an dem Menschen angebracht, wobei die weiteren Beschleunigungssensoren Beschleunigungen weiterer Körperteile des Menschen 100 erfassen können und die weiteren Geräuschsensoren beispielsweise Umgebungsgeräusche bzw. Geräusche von anderen Organen (Herz) erfassen können.

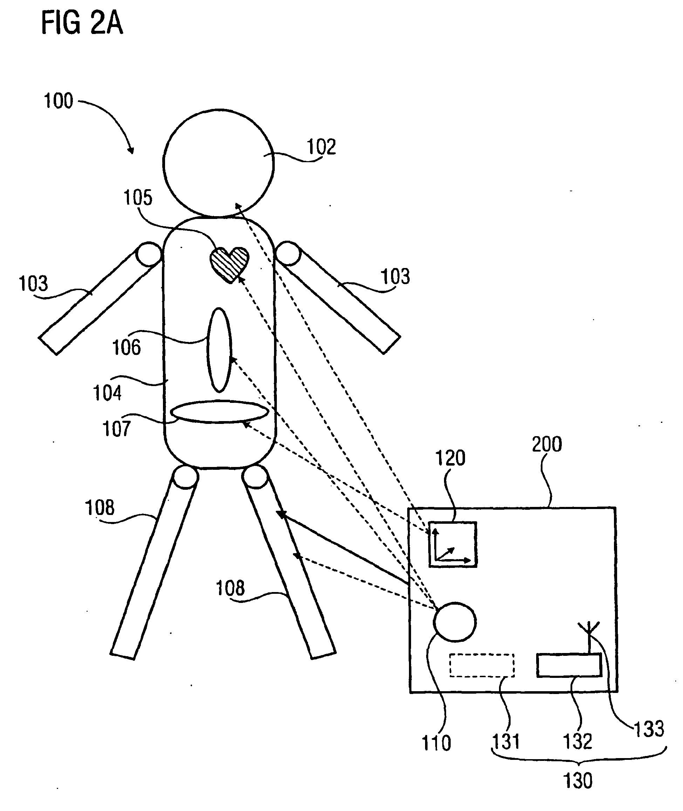

Fig. 2A zeigt den Menschen 100 mit einem Kopf-Halsbereich 102, zwei Armen 103, einem Torso 104 mit einem Herzen 105, einem Wirbelsäulenbereich 106, einem Hüftbereich 107 und zwei Beinen 108. An dem Menschen 100 können an verschiedenen Körperregionen oder Körperstellen der Geräuschsensor 110 und der Beschleunigungssensor 120 angebracht sein (dargestellt durch gestrichelte Linien). Sofern der Beschleunigungssensor 120 und der Geräuschsensor 110 in einem Kombisensor 200 untergebracht sind, kann dieser Kombisensor beispielsweise an einem Bein oder Oberschenkel 108 angebracht sein (durchgezogene Linie). Die Auswerteeinheit 130 kann entweder in dem Geräuschsensor 110 oder in dem Beschleunigungssensor 120 angeordnet sein und beispielsweise einen Mikrocontroller 131 (eine Intelligenz), eine Übertragungseinheit 132 (beispielsweise basierend auf Bluetooth) und eine Antenne 133 aufweisen. Die Auswerteeinheit kann ebenfalls in dem Kombisensor 200 bzw. in deren Gehäuse angeordnet sein.

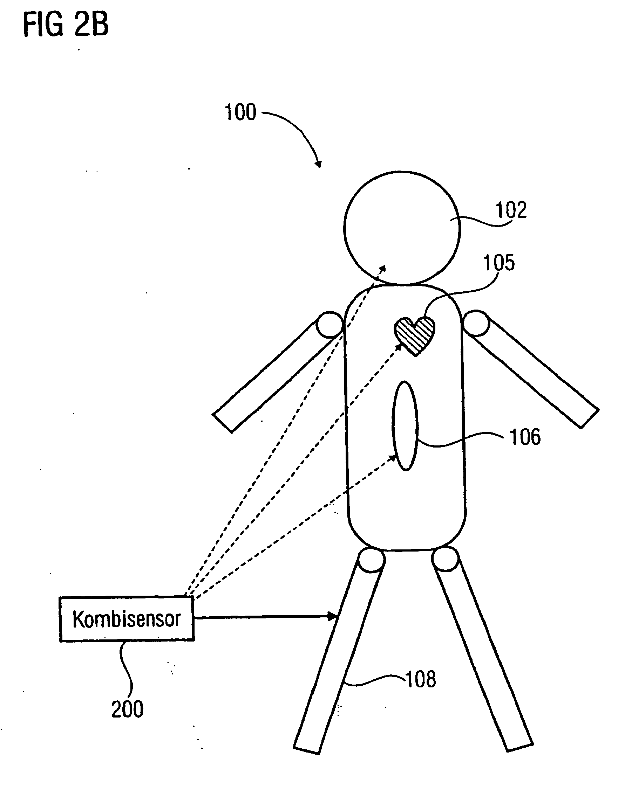

Fig. 2B zeigt mögliche Fixierungsstellen des Kombisensors 200, die beispielsweise an einem Bein 108 oder dem Oberschenkel angeordnet sein kann. Darüber hinaus ist es ebenfalls möglich, den Kombisensor 200 an einer anderen Körperstelle anzuordnen, sofern sich dies für die Erfassung der Daten als günstig erweist, beispielsweise an einer Wirbelsäulenregion 106 zur Erfassung der Geräusche der Stützmuskulatur oder in der Nähe des Herzens 105 zur Erfassung von Herzgeräuschen oder in dem Hals-Kopfbereich 102, beispielsweise zur Erfassung von Ermüdungserscheinungen (Kopfnicken). Diese alternativen Anordnungen sind mit gestrichelten Linien gekennzeichnet.

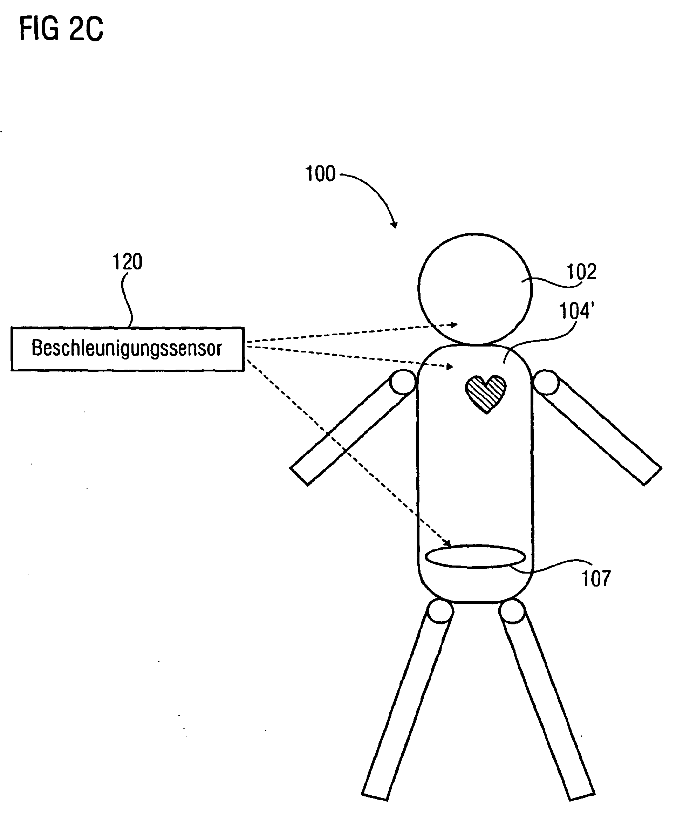

Fig. 2C zeigt mögliche Fixierungsorte des Beschleunigungssensors 120. Diese Positionen beziehen sich insbesondere auf die Möglichkeit, wenn der Beschleunigungssensor 120 und der Geräuschsensor 110 jeweils getrennt an verschiedenen Körperstellen angeordnet sind. Der Beschleunigungssensor 120 kann beispielsweise in dem Hüftbereich 107, dem Hals-Kopfbereich 102 oder einem Schulterbereich 104' angeordnet sein.

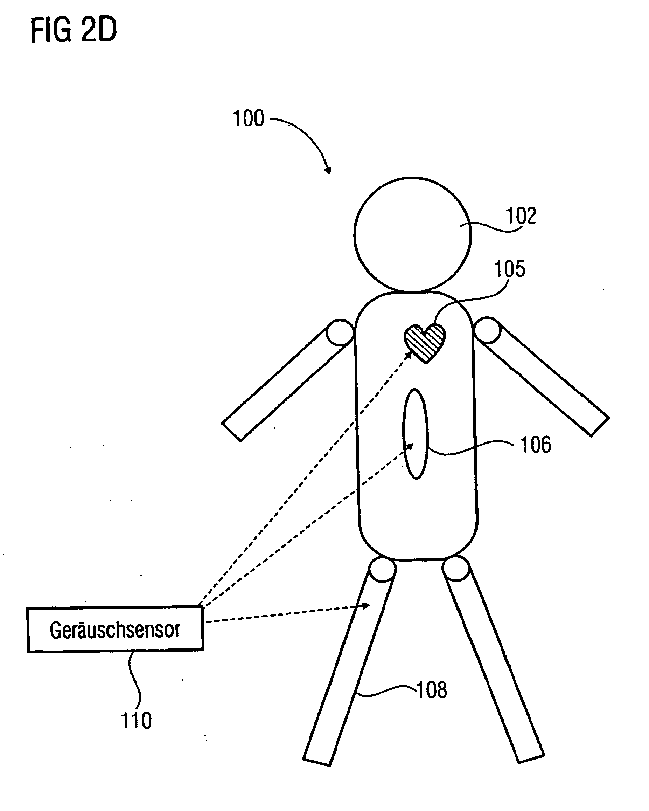

Fig. 2D zeigt mögliche Anordnungen des Geräuschsensors 110, wobei wie in Fig. 2C bei diesem Ausführungsbeispiel davon ausgegangen wird, dass der Geräuschsensor 110 und der Beschleunigungssensor 120 getrennt voneinander am menschlichen Körper 100 fixiert werden. Mögliche Anordnungen des Geräuschsensors 110 sind dabei der Oberschenkel oder das Bein 108, der Wirbelsäulenbereich 106, nahe dem Herzen 105 oder weitere, nicht in der Fig. 2D gezeigte Bereiche des menschlichen Körpers (beispielsweise des Kopf-Halsbereiches 102).

Bei den Ausführungsbeispielen von Fig. 2C und 2D kann die Auswerteeinheit 130 entweder in dem Geräuschsensor 110 oder dem Beschleunigungssensor 120 angeordnet sein bzw. in deren Gehäuse untergebracht werden. Somit sind ebenfalls die Untermodule, Mikrocontroller 131 ("Intelligenz") und die Übertragungseinheit 132 (Bluetooth) im Falle von Einzelsensoren gemäß Aspekten der Ergonomie in allen der Sensoren bzw. im Falle eines Kombisensors in dessen Gehäuse integriert. Um miteinander kommunizieren zu können, brauchen die getrennt angeordneten Sensoren (Geräuschsensor 110 und Beschleunigungssensor 120) jeweils eine Übertragungseinheit 132, damit die Daten ausgetauscht werden können. Die Übertragung kann drahtgebunden oder auch drahtlos geschehen. Andererseits kann es ausreichend sein, wenn der Mikrocontroller 131 nur in einen der Sensoren (oder deren Gehäuse) integriert ist. Es ist ebenfalls möglich unter Verwendung der Übertragungseinheiten 132, die Daten an eine externe Einheit zu übermitteln, wobei die externe Einheit ebenfalls am Menschen oder einen anderen Platz (auch weiter entfernt vom Menschen) fixiert sein kann. Durch Verwendung von mehreren Beschleunigungssensoren ist es darüber hinaus möglich, die Messgenauigkeit zu erhöhen, wobei Beschleunigungssensoren beispielsweise in dem Hüftbereich 107 und in dem Schulterbereich 104' angeordnet sein können.

Zum Anbringen oder zum Fixieren kann der Kombisensor 200 oder der Beschleunigungssensor 120 und/oder der Geräuschsensor 110 und/oder die Auswerteeinheit 130 eine Einrichtung zur Fixierung oder eine Einrichtung zum Anbringen aufweisen, die beispielsweise ihrerseits einen Klettverschluss, Kleber, Gummihalterung aufweisen.

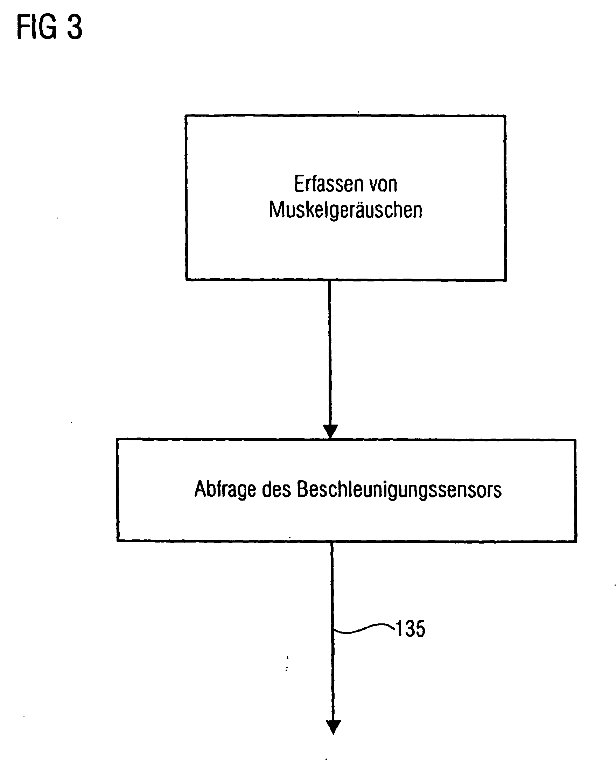

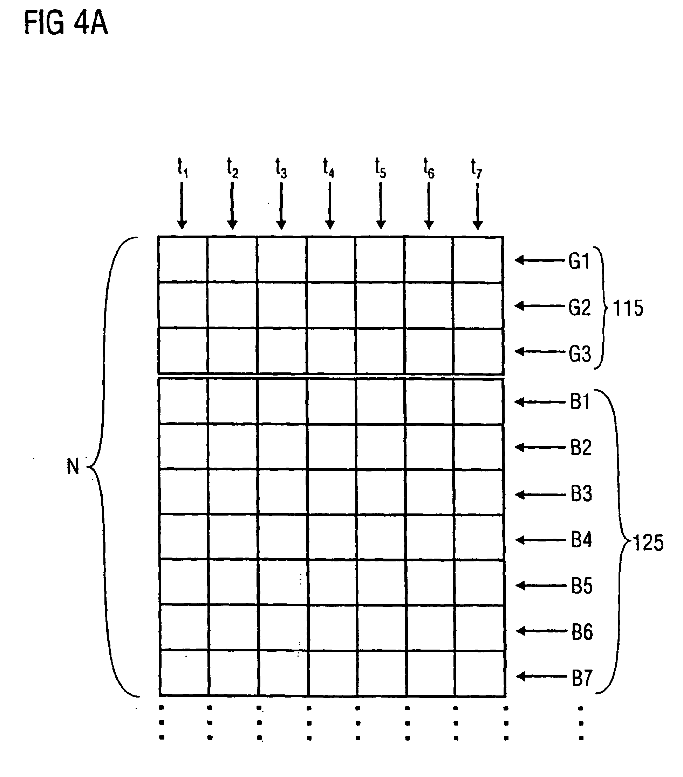

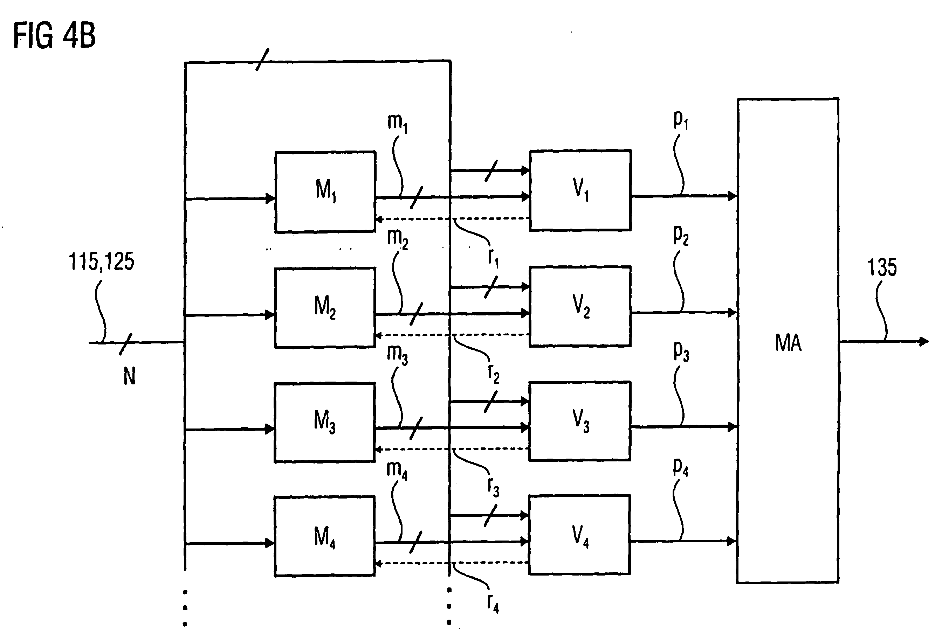

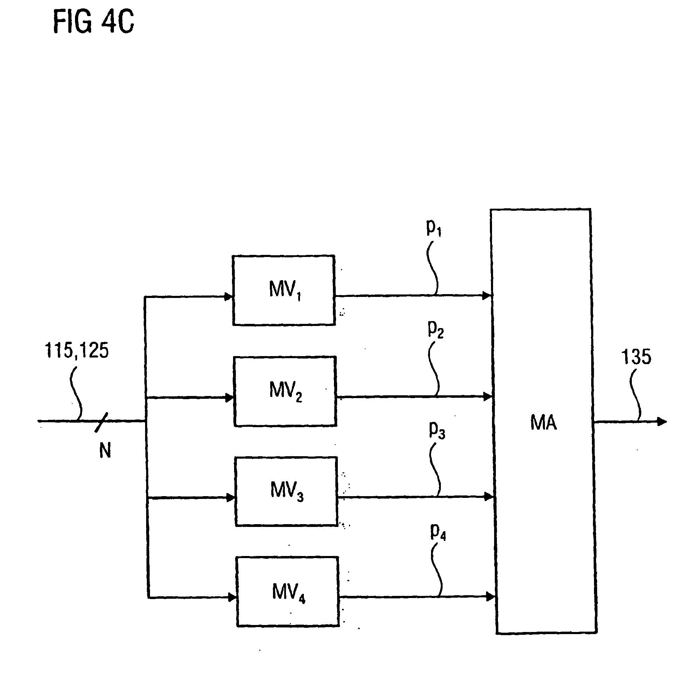

Im Folgenden werden verschiedene Ausführungsbeispiele beschrieben, wie die Datenerfassung und -auswertung erfolgen kann. In Fig. 3 wird zunächst die einfachste Möglichkeit beschrieben, bei der zunächst nur Geräuschdaten erfasst werden, währenddessen die Fig. 4 eine parallele Erfassung und Auswertung beschreibt, die zwar aufwendiger ist, jedoch eine Reihe von Vorteilen bietet.

Fig. 3 zeigt ein Ausführungsbeispiel für ein Verfahren, bei dem beispielsweise ständig (kontinuierlich) oder in regelmäßigen Zeitabständen die Muskelgeräusche erfasster werden und nur wenn Besonderheiten, die mit einem Tonusverlust einhergehen, auftreten, wird ein Abfragesignal 115' generiert. Als Folge wird ein Abfragen des Beschleunigungssensors 120 vorgenommen und nur wenn der Beschleunigungssensor 120 ebenfalls Besonderheiten zum Beispiel innerhalb eines vorbestimmten Zeitintervalls (Zeitfenster) wie beispielsweise Torkeln, Kopfnicken feststellt, erfolgt die Ausgabe des Warnsignals 135 oder der Ereignisdaten. Das Zeitfenster kann beispielsweise 0,1 Sekunden, 0,5 Sekunden, 1 Sekunde oder 5 Sekunde umfassen. Sofern innerhalb des Zeitfenster der Beschleunigungssensor 120 keine Besonderheiten feststellt, kann zum Beispiel das Geräuschsignal ignoriert werden.

Die Fig. 4A bis 4C beschreiben mögliche Ausführungsbeispiele für die Auswerteeinheit 130, wobei die Fig. 4A zunächst die Datenstruktur beschreibt, mit der die Auswerteeinheit 130 Daten analysieren und entsprechende Schlussfolgerungen ziehen kann (Erzeugung eines Warnsignals, Benachrichtigung einer Benachrichtigungsstelle, etc.).

Die Auswerteeinheit 130 erhält zunächst die Beschleunigungsdaten 125 von dem oder die Beschleunigungssensor(en) 120, die sich auf mehrere Beschleunigungen (z.B. Beschleunigungen entlang unterschiedlicher Raumrichtungen) oder aber auf Beschleunigungen bezüglich verschiedener Beschleunigungssensoren 125 beziehen können. In Fig. 4A ist ein Beispiel gezeigt, bei dem sieben Beschleunigungsdaten 125 erfasst werden, von denen beispielsweise drei Beschleunigungswerte B1, B2, B3 sich auf die drei Raumrichtungen der Beschleunigung beispielsweise der Hüfte, weitere drei Beschleunigungswerte B4, B5, B6 auf die drei Raumrichtungen der Beschleunigung der Schulterregion 102 und drei weitere Beschleunigungen B7, B8, B9 (B8 und B9 sind nicht in der Fig. 4A gezeigt) sich auf die drei Raumrichtungen der Beschleunigung des Oberschenkels 108 beziehen. Die hier angegebenen Beschleunigungsdaten stellen nur Beispiele dar und im Allgemeinen können für jeden Beschleunigungssensor 125 auch nur eine oder zwei oder alle drei Beschleunigungen (bzgl. aller drei Raumrichtungen) erfasst werden. Ebenso können weitere oder weniger Beschleunigungssensoren 125 verwendet werden. Ferner umfasst der Datensatz, wie in Fig. 4A gezeigt, drei Geräuschdaten G1, G2, G3, die beispielsweise einem Geräusch des Oberschenkelmuskels 108, des Herzmuskels 105 und des Umgebungsgeräusches entsprechen können.

Im Allgemeinen werden N Daten erfasst werden, wobei bei weiteren Ausführungsbeispielen mehr oder weniger als die gezeigten Daten erfasst werden können. Dadurch kann entweder eine genauere Vorhersage oder Detektion von kritischen Zuständen erfolgen oder andererseits (beim Erfassen von weniger Daten) kann der Rechenaufwand der Auswerteeinheit 130 verringert werden.

Die Geräusch- und Beschleunigungsdaten 115, 125 können weiterhin zu verschiedenen Zeitpunkten erfasst werden. Zum Beispiel kann ein Satz von Daten zu einer ersten Zeit t₁, einer zweiten Zeit t₂, zu einer dritten Zeit t₃, ... bis zu einer siebten Zeit t₇ erfasst werden, wobei der Zeitrhythmus oder Taktrhythmus, in dem Daten erfasst werden, an die gegebene Situation angepasst werden kann. Beispielsweise können Daten im Sekundentakt oder aber mit einem Takt in einem Bereich zwischen 1/15 bis 2 Sekunden erfasst werden. Bei weiteren Ausführungsbeispielen kann dieser Takt variabel einstellbar sein, so dass angepasst an die gegebene Situation (schnellere oder langsamere Bewegung), eine effiziente Feststellung eines kritischen Zustands ermöglicht wird.

In Fig. 4B ist gezeigt, wie die Geräuschdaten 115 und Beschleunigungsdaten 125 in der Auswerteeinheit 130 verarbeitet werden können, wobei die Anzahl der Daten die zu einem gegebenen Zeitpunkt erfasst werden mit N bezeichnet ist. Bei dem Ausführungsbeispiel von Fig. 4B werden zunächst die Geschwindigkeits- und Beschleunigungsdaten 115, 125 in eine erste Modelleinheit M₁, in eine zweite Modelleinheit M₂, in eine dritte Modelleinheit M₃, in eine vierte Modelleinheit M₄ eingegeben und die Modelleinheiten Mᵢ (i = 1, 2, 3, 4, ...) erfassen beispielsweise die Daten in der Art und Weise, wie es in der Fig. 4A dargestellt ist, das heißt z.B. in einem vorgegeben Takt werden zu vorgegebenen Zeiten tₙ Daten erfasst, so dass sich die in der Fig. 4A gezeigte Matrix, bei der die verschiedenen Daten in verschiedene Zeilen geschrieben werden, und Daten, die zu verschiedenen Zeitpunkte erfasst werden, in verschiedenen Spalten angeordnet sind.

Den verschiedenen Modelleinheiten Mi liegen verschiedene Modelle zugrunde, wobei sich die Modelle auf verschiedene Tätigkeiten des Menschen beziehen können, die beispielsweise ein Gehen, ein Treppensteigen, ein Laufen oder Radfahren oder andere Tätigkeiten umfassen können. Ferner kann ein Modell sich auf einen Ernstfall beziehen, das heißt, ein typisches Muster eines Sturzes simulieren. Mit Hilfe der Modelle kann nun unter Zugrundelegung der Daten zu den beispielhaften Zeiten t₁ bis tₙ ein Datensatz für die nächstfolgende Zeit tₙ₊₁ berechnet werden. Dies kann beispielsweise über eine Faltung geschehen. Der vorausberechnete Datensatz entspricht dabei dem wahrscheinlichsten Zustand, den das System unter Verwendung des entsprechenden Modells in der Folgezeit tₙ₊₁ einnehmen wird. Diese so vorausberechneten oder geschätzten Daten mᵢ (i = 1, 2, 3, 4, ...) werden in Vergleichseinheiten Vᵢ eingegeben, wobei die Vergleichseinheit Vᵢ die vorausberechneten Daten mᵢ, die sich auf die Zeit tₙ₊₁ beziehen, mit den tatsächlich gemessenen Daten zu der Zeit tₙ₊₁ vergleicht. Die Vergleichseinheiten Vᵢ liefern dann als Ausgabe Prozentwerte pᵢ, mit denen das zugrunde gelegte Modell mit den tatsächlich gemessenen Werten übereinstimmt. Die so erhaltenen Prozentwerte pᵢ werden anschließend in einer Modellauswahleinheit MA eingelesen und die Modellauswahleinheit MA wählt anhand der erhaltenen Prozentzahlen pᵢ jenes Modell aus, das die höchste prozentuale Übereinstimmung mit den tatsächlich gemessenen Werten aufweist.

Ein gegebenes Modell, wie beispielsweise ein Gehen des Menschen 100, kann beispielsweise mit unterschiedlicher Geschwindigkeit geschehen. Demzufolge ist es sinnvoll, dass die Vergleichseinheiten Vᵢ eine Rückkopplung aufweisen, um das verwendete Modell beziehungsweise ein Parameter des verwendeten Modells entsprechend zu ändern. Diese Rückkopplung kann über die Rückkoppeldaten rᵢ erfolgen und einen Parameter enthalten, (zeitliche Streckung oder zeitliche Stauchung beispielsweise) mit dem das zugrundegelegte Modell verändert wird. Dieser Parameter rᵢ kann durch die Vergleichseinheit Vᵢ beispielsweise dadurch ermittelt werden, dass die Vergleichseinheit Vᵢ eine Optimierung hinsichtlich der dem Modell zugrunde gelegten Parameter durchführt, so dass die prozentuale Übereinstimmung pᵢ maximiert wird. Für das gegebene Beispiel des Gehens eines Menschen 100 kann dies so geschehen, dass die Gehgeschwindigkeit des Menschen 100 durch die Vergleichseinheit Vᵢ so lange variiert wird, bis die prozentuale Übereinstimmung pᵢ einen maximalen Wert aufweist.

Die Fig. 4C zeigt ein weiteres Ausführungsbeispiel für die Auswertung der Geräusch- und Beschleunigungsdaten 115, 125. Es wird wieder davon ausgegangen, dass der Datensatz insgesamt N-Daten enthält, die nach einander zu gegebenen Zeitpunkten tₙ (n = 1, 2, 3, ...) erfasst werden. Diese Datensätze werden bei dem Ausführungsbeispiel, wie es in der Fig. 4C gezeigt ist, in Modelltesteinheiten MVᵢ eingegeben, wobei bei diesem Ausführungsbeispiel angenommen wurde, dass vier Modelltesteinheiten MVᵢ vorhanden sind, deren Anzahl jedoch bei weiteren Ausführungsbeispielen variieren kann. Die Modelltesteinheiten MVᵢ erfassen nun die Geräusch- und Beschleunigungsdaten 115, 125 wiederum in der Art, wie es in der Fig. 4A gezeigt ist, so dass eine Matrix entsteht mit N Zeilen und n Spalten, wobei Daten verschiedener Art in verschiedene Zeilen und Daten zu verschiedenen Zeitpunkten in verschiedenen Spalten der Matrix angeordnet sind. Die so erhaltene Matrix von Daten weist nun für jede Tätigkeit oder Bewegung des Menschen 100 ein charakteristisches Muster auf (wobei wiederum das charakteristische Muster zeitlich gestaucht oder gestreckt sein kann).

Die Modelltesteinheiten MVᵢ testen nun das erfasste Datenmuster mit den verschiedenen Modellen, d.h. die Modelltesteinheit MV₁ vergleicht die erfassten Daten (das heißt die Daten in der Matrix, wie es in der Fig. 4A dargestellt ist) mit dem ersten Modell, die zweite Modelltesteinheit MV₂ testet den Datensatz mit dem zweiten Modell, usw. Im Allgemeinen werden die Modelltesteinheiten MVᵢ keine 100 %ige Übereinstimmung der gemessenen Datensätze oder der Datenmatrix aus Fig. 4A mit der den Modellen zugrunde gelegten Datenstrukturen erreichen und die Modelltesteinheiten MVᵢ einen prozentualen Übereinstimmungswert pᵢ ausgibt. Der prozentuale übereinstimmung.swert pᵢ gibt an, zu welchem Prozentsatz die tatsächlich gemessenen Daten oder die Datenmatrix mit dem zugrunde gelegten Modell oder Modellen übereinstimmt. Die Modelltesteinheiten MVᵢ können dabei wiederum intern Parameter des zugrunde gelegten Modells mit dem Ziel variieren, dass die erhaltenen prozentualen Übereinstimmungswerte pᵢ maximal sind. Die erhaltenen prozentualen Übereinstimmungswerte pₑ werden wiederum in einer Modellauswahleinheit MA eingegeben, wobei die Modellauswahleinheit MA wiederum das Modell auswählt, dass eine möglichst gute Übereinstimmung der gemessenen Datenmatrix mit dem zugrunde gelegten Modell aufweist.

Die parallele Erfassung der Daten beziehungsweise der parallele Abgleich der erfassten Daten mit den zugrunde gelegten Modellen, erlaubt es nun, dass ein Wechsel der Tätigkeit des Menschen 100 beispielsweise vom Gehen zum Treppensteigen oder zum Hinsetzen sich dadurch zeigt, dass die prozentualen Übereinstimmungswerte pᵢ sich plötzlich ändern, so dass die Modellauswahleinheit MA das zugrunde gelegte Modell dynamisch anlassen kann. Wenn die Modellauswahleinheit MA jedoch ein Datenmuster erkennt, das auf einen kritischen Zustand hindeutet (beispielsweise das Modell Sturz oder Schwindel) kann die Modellauswahleinheit MA ein Warnsignal 135 ausgeben, beziehungsweise eine entsprechende Benachrichtigung einer Notfallstelle (Benachrichtigungsstelle) übermitteln. Bezogen auf einen Fahrzeugführer kann die Modellauswahleinheit MA beim Feststellen eines Zustandes (oder Modells), das auf eine übermüdung des Fahrzeugführers hindeutet (beispielsweise Kopfnicken), ein entsprechendes Warnsignal an den Fahrzeugführer ausgeben, um ihn auf seinen kritischen Zustand hinzuweisen.

Bei der Auswertung der Datensätze oder der Datenmatrix in der Auswerteeinheit 130 kann beispielsweise ein Kalmanfilter zur Anwendung kommen. Basierend auf vorgegeben Modellen nutzt ein Kalmanfilter eine Zustandsgleichung mit deren Hilfe zukünftige Zustände des Systems abgeschätzt werden können. Dies ist insbesondere dahingehend sinnvoll, da ein Kalmanfilter die Möglichkeit bietet, entsprechende Fehler oder Rauschen herauszufiltern und es ferner für Echtzeitanwendungen prädestiniert ist. Ferner ist es bei einem Kalmanfilter möglich, über Parameteränderungen das Modell dynamisch zu ändern, um dadurch eine bessere Übereinstimmung der geschätzten Zustände von den tatsächlichen Zuständen zu erreichen.

Bei weiteren Ausführungsbeispielen kann es sinnvoll sein, dass nicht alle Modelle parallel mit einer gleichen Intensität bearbeitet werden, sondern dass über Zeiträumen, in denen sich ein gegebenes Modell die Situation sehr gut beschreibt, die anderen Modelle nur in größeren Zeitabständen getestet werden und dass erst bei einem möglichen Modellwechsel, aufgrund stark sinkender Übereinstimmungswahrscheinlichkeiten pᵢ die alternativen Modelle wieder mit voller Intensität getestet werden. Es ist auch möglich, zu einer gegebenen Zeit immer nur ein Modell hinsichtlich der Übereinstimmung mit den tatsächlich gemessenen Werten zu testen und nur dann, wenn die Ubereinstimmungswahrscheinlichkeiten pᵢ unterhalb einer bestimmten Schwelle liegen, andere Modelle nacheinander durchgetestet werden. Dies kann solange geschehen, bis sich wieder ein dominierendes Modell herausstellt. Bei weiteren Ausführungsbeispielen passt die Auswerteeinheit 130 die Modelle den natürlichen Eigenheiten des Menschen 100 an. Damit passt sich nach einiger Zeit mittels einer derartigen Intelligenz jedes der Modelle (zum Beispiel Gehen, Treppensteigen, Stehen, Liegen usw.) individuell an den Menschen 100 an.

Somit beziehen sich Ausführungsbeispiele der vorliegenden Erfindung insbesondere auf ein Verfahren und eine Vorrichtung zur Feststellung eines Tonusverlustes bzw, eines bevorstehenden Tonusverlustes einer Muskulatur eines Menschen. Ausführungsbeispiele weisen verschiedene Kombinationen von Messwertaufnehmern (Geräuschsensoren 110 und Beschleunigungssensoren 120) auf, die einzeln oder auch mehrere an verschiedenen Körperstellen angeordnet sein können und ferner bieten Ausführungsbeispiele ein Verfahren zur Feststellung eines Schwindelzustandes oder einer Ermüdungserscheinung oder anderer zu einem bevorstehenden Tonusverlustes führende Zustände des menschlichen Körpers, wobei ein entsprechendes algorithmisches Verfahren verwendet werden kann. Das entsprechende algorithmische Verfahren setzt dabei beispielsweise ein Zusammentreffen von zwei Ereignissen voraus, zum einen ein Auftreten von entsprechenden Muskelgeräuschen und zum anderen ein Erfassen einer entsprechenden Beschleunigung (einem Beschleunigungsgrenzwert oder Musters), beispielsweise in Folge eines Sturzes, Kopfnickens oder anderer plötzlicher Körperbewegungen. Die entsprechenden Muskelgeräusche können beispielsweise einem Geräuschgrenzwert oder einem Verlassen einer Bandbreite (z.B. in der Frequenzdarstellung) des Geräuschmusters entsprechen. Die Vorrichtung ist somit sensitiv in bezug auf stark erhöhte oder stark verringerte Muskelgeräuschen. Der Beschleunigungsgrenzwert kann beispielsweise einem Aufprall des menschlichen Körpers 100 oder anderer abrupter Körperbewegungen (Kopfnicken) entsprechen.

Im Gegensatz zu konventionellen Verfahren oder Vorrichtungen, die nur auf der Erfassung von Beschleunigungsdaten basieren, bieten Ausführungsbeispiele der vorliegenden Erfindung damit ein erhöhtes Maß an Sicherheit und Zuverlässigkeit hinsichtlich der Detektion entsprechend krankhafter oder unerwünschter Zustände des menschlichen Körpers.

Insbesondere wird darauf hingewiesen, dass abhängig von den Gegebenheiten das erfindungsgemäße Schema auch in Software implementiert sein kann. Die Implementierung kann auf einem digitalen Speichermedium oder einem nichtflüchtigen Flashspeicher, insbesondere einer Diskette oder einer CD mit elektronisch auslesbaren Steuersignalen erfolgen, die so mit einem programmierbaren Computersystem zusammenwirken können, dass das entsprechende Verfahren ausgeführt wird. Allgemein besteht die Erfindung somit auch in einem Computerprogrammprodukt mit auf einem maschinenlesbaren Träger gespeicherten Programmcode zur Durchführung des erfindungsgemäßen Verfahrens, wenn das Computerprogrammprodukt auf einem Rechner abläuft. In anderen Worten ausgedrückt kann die Erfindung somit als ein Computerprogramm mit einem Programmcode zur Durchführung des Verfahrens realisiert werden, wenn das Computerprogramm auf einem Computer oder einem so genannten embedded system abläuft.

## Patentansprüche

1. Vorrichtung zur Vorhersage eines Verlustes einer Kontrolle über einen Muskel eines Menschen (100), mit:
einer Einrichtung (110) zum Detektieren eines Muskelgeräusches;
einer Einrichtung (120) zur Erfassung einer Beschleunigung des Menschen (100); und
einer Einrichtung (130) zum Auswerten des Geräusches und der Beschleunigung, um aus dem Geräusch und der Beschleunigung einen bevorstehenden Verlust der Kontrolle über den Muskel festzustellen.

2. Vorrichtung nach Anspruch 1, bei der die Einrichtung (110) zum Detektieren ausgebildet ist, um das Geräusch eines Stützmuskels oder eines Beinmuskels zu detektieren und bei der die Einrichtung (130) zum Auswerten ausgebildet ist, um einen bevorstehenden Kontrollverlust des Stützmuskels oder des Beinmuskels festzustellen.

3. Vorrichtung nach Anspruch 1 oder Anspruch 2, bei der die Einrichtung (130) zum Auswerten ausgebildet ist, im Falle eines Feststellens eines bevorstehenden Verlustes der Kontrolle der Muskulatur, ein Warnsignal (135) auszugeben, das von dem Menschen (100) erfassbar ist.

4. Vorrichtung nach Anspruch 3, bei der das Warnsignal (135) ein Lichtsignal und/oder ein akustisches Signal und/oder einen fühlbaren Reiz umfasst.

5. Vorrichtung nach Anspruch 3 oder Anspruch 4, bei der die Einrichtung (130) zum Auswerten ausgebildet ist, das Warnsignal (135) an eine vorbestimmte Benachrichtigungsstelle zu übermitteln.

6. Vorrichtung nach einem der vorhergehenden Ansprüchen, bei der die Einrichtung (130) zum Auswerten ausgebildet ist, das Geräusch und die Beschleunigung in zeitlichen Abständen zu erfassen und zu einem Datensatz zu kombinieren.

7. Vorrichtung nach Anspruch 6, bei der die Einrichtung (130) zum Auswerten ausgebildet ist, den Datensatz mit einem Modelldatensatz zu vergleichen und einen prozentualen Obereinstimmungswert zu ermitteln, um auf eine aktuelle Bewegung des Menschen (100) zu schließen, wobei der Modelldatensatz einem Datensatz, der durch eine bestimmte Bewegung des Manchen (100) erzeugbar ist, entspricht.

8. Vorrichtung nach Anspruch 7, wobei der Modelldatensatz einen Parameter aufweist und bei dar die Einrichtung (130) zum Auswerten ausgebildet ist, den Parameter zu variieren, um einen prozentualen Übereinstimmungswert zu maximieren. ,

9. Vorrichtung nach einem der vorhergehenden Ansprüche, bei der die Einrichtung (130) zum Auswerten ausgelegt ist, Daten der Einrichtung (110) zum Detektieren eines Geräusches und/oder Daten der Einrichtung (120) zum Erfassen einer Beschleunigung abzuspeichern.

10. Vorrichtung nach einem der vorhergehenden Ansprüche, bei der die Einrichtung (130) zum Auswerten eine Obertragungseinrichtung (132) aufweist und die Übertragungseinrichtung (132) ausgebildet ist, Daten von der Einrichtung (110) zum Detektieren eines Geräusches und/oder Daten von der Einrichtung (120) zur Erfassung einer Beschleunigung zu erfassen oder an eine Zentraleinheit zu übermitteln.

11. Vorrichtung nach Anspruch 10, bei der die Übertragungseinrichtung (132) Daten drahtlos erfasst oder drahtlos überträgt.

12. Vorrichtung nach einem der vorhergehenden Ansprüche, bei der die Einrichtung (110) zum Detektieren und die Einrichtung (120) zur Erfassung und die Einrichtung (130) zum Auswerten in einem kombinierten Sensor (200) gemeinsam gehäust sind.

13. Vorrichtung nach Anspruch 12, bei der der kombinierte Sensor (200) eine Einrichtung zum Anbringen an einem Oberschenkel oder Bein (108) des Menschen (100) aufweist.

14. Vorrichtung nach einem der vorhergehenden Ansprüche, bei der die Einrichtung (110) zum Detektieren eine Einrichtung zum Anbringen an einem Oberschenkel (108) und die Einrichtung (120) zur Erfassung einer Beschleunigung eine Einrichtung zum Anbringen an einem Hüftbereich (10'7) des Menschen (100) aufweisen.

15. Vorrichtung nach einem der vorhergehenden Ansprüche, die ferner eine weitere Einrichtung zur Erfassung von Herzmuskelgeräuschen aufweist und die weitere Einrichtung nahe dem Herzen (105) des Menschen (100) anbringbar ist und die Einrichtung zum Auswerten (130) ausgebildet ist, um die Feststellung des Verlustes der Kontrolle auch aus den Herzmuskelgeräuschen vorzunehmen.

16. Vorrichtung nach einem der vorhergehenden Ansprüche, bei der die Einrichtung (110) zum Detektieren und die Einrichtung (120) zur Erfassung oder der Kombisensor (200) eine Einrichtung zum Anbringen an einer Hals-Kopfregion (102) aufweisen, um ein Kopfnicken in Folge einer Übermüdung des Menschen (100) festzustellen.

17. Vorrichtung nach einem der vorhergehenden Ansprüche, bei der die Einrichtung (110) zum Detektieren und/oder die Einrichtung (120) zur Erfassung eine drahtlose Sendeeinrichtung aufweist, wobei die drahtlose Sendeeinrichtung ausgelegt ist, Sensordaten an die Einrichtung (130) zum Auswerten zu übermitteln.

18. Vorrichtung nach einem der vorhergehenden Ansprüche, bei der die Einrichtung (130) eine Datenübertragung mittels Bluetooth ermöglicht.

19. Vorrichtung nach einem der vorhergehenden Ansprüche, die ferner eine weitere Einrichtung zum Detektieren weiterer Geräusche, die ihren Ursprung außerhalb des Menschen (100) haben, aufweist, und wobei die Einrichtung (130) zum Auswerten ausgelegt ist, Daten von der weiteren Einrichtung zur Detektieren weiterer Geräusche zu empfangen und eine Geräuschdifferenz zwischen den Geräuschen und den weiteren Geräusche zu bilden.

20. Vorrichtung nach einem der vorhergehenden Ansprüche, bei der die Einrichtung (120) zur Erfassung einer Beschleunigung ausgelegt ist, einen zeitlichen Verlauf einer Lage eines Torsos (104) des Menschen (100) relativ zum Gravitationsfeld zu bestimmen.

21. Vorrichtung nach einem der vorhergehenden Ansprüche, bei der die Einrichtung (130) zum Auswerten ausgelegt ist, Daten der Einrichtung (110) zum Detektieren eines Geräusches in vorbestimmten Zeitintervallen zu empfangen und ferner ausgelegt ist, bei Erreichen eines Geräuschgrenzwertes zusätzlich Daten von der Einrichtung (120) zur Erfassung einer Beschleunigung zu erhalten, um daraus bei überschreiten eines Beschleunigungsgrenzwertes ein Warnsignal (135) zu erzeugen.

22. Vorrichtung nach Anspruch 21, bei dem der Geräuschgrenzwert eine Bandbreite eines Geräuschmusters darstellt und bei dem der Beschleunigungsgrenzwert eine Verletzung eines bestimmten Beschleunigungswertes umfasst.

23. Vorrichtung nach Anspruch 21 oder Anspruch 22, bei der die Einrichtung (130) zum Auswerten ausgelegt ist, Beschleunigungsdaten von der Einrichtung (120) zur Erfassung der Beschleunigung innerhalb eines vorbestimmten Zeitintervalls nach dem die Einrichtung (110) zum Detektieren ein Geräusch oberhalb oder unterhalb des Geräuschgrenzwertes detektiert hat, zu erfassen und bei Überschreiten des Beschleunigungsgrenzwertes das warnsignal (135) zu erzeugen.

24. Verfahren zur Vorhersage eines Verlustes einer Kontrolle über einen Muskel eines Menschen (100), mit:
Detektieren eines Muskelgeräusches;
Erfassen einer Bschleunigung des Menschen; und
Auswerten des Geräusches und der Beschleunigung, um aus dem Geräusch und der Beschleunigung einen bevorstehenden Verlust der Kontrolle über die Muskulatur festzustellen.

25. Verfahren nach Anspruch 24, wobei der Schritt des Detektierens des Geräusches an einer ersten Körperstelle und der Schritt des Erfassens der Beschleunigung an einer zweiten Körperstelle erfolgt und das Verfahren ferner einen Schritt eines Optimierens aufweist, wobei der Schritt des Optimierens einer Veränderung der ersten und/oder der zweiten Körperstelle umfasst, so dass ein verbessertes Detektieren des Geräusches und ein verbessertes Erfassen der Beschleunigung erreicht wird.

26. Verfahren nach Anspruch 25, bei dem der Schritt des Optimierens dahin gehend durchgeführt wird, dass Geräusche, die einen möglichen Tonusverlust anzeigen, klar detektierbar sind.

27. Verfahren nach einem der Ansprüche 24 bis 26, das ferner:
ein Detektieren von Umgebungsgeräuschen; und
ein Bilden eines Differenzsignals aufweist,
wobei eine Quelle der Umgebungsgeräusche außerhalb des Muskels liegen und einen vom Tonusverlust abweichenden Ursprung aufweisen, und das Differenzsignal aus dem Geräusch des Muskels und dem Umgebungsgeräusch gebildet wird, so dass eine Sensitivität für Muskelgeräusche infolge eines bevorstehenden Tonusverlustes erhöht wird.

28. Computerprogramm mit einem Programmcode zum Durchführen des Verfahrens nach Anspruch 24, wenn das Programm auf einer Vorrichtung gemäß Anspruch 1 abläuft.

## Claims

1. A device for predicting a loss of control over a muscle of a human being (100), comprising:
means (110) for detecting a muscle sound ;
means (120) for acquiring an acceleration of the human being (100); and
means (130) for evaluating the sound and the acceleration to determine an imminent loss of control of the muscle from the sound and the acceleration.

2. The device in accordance with claim 1, wherein the means (110) for detecting is implemented to detect the sound of a supportive muscle or a leg muscle, and wherein the means (130) for evaluating is implemented to determine an imminent loss of control of the supportive muscle or the leg muscle.

3. The device in accordance with claims 1 or 2, wherein the means (130) for evaluating is implemented to output, in the case of determining an imminent loss of control of the muscular system, a warning signal (135) detectable by the human being (100).

4. The device in accordance with claim 3, wherein the warning signal (135) comprises a photo signal and/or an acoustic signal and/or a tangible stimulus.

5. The device in accordance with claims 3 or 4, wherein the means (130) for evaluating is implemented to transfer the warning signal (135) to a predetermined notification institution.

6. The device in accordance with one of the preceding claims, wherein the means (130) for evaluating is implemented to acquire the sound and the acceleration in temporal intervals and combine same to form a data set.

7. The device in accordance with claim 6, wherein the means (130) for evaluating is implemented to compare the data set to a model data set and to determine a percentage matching value to draw conclusions about a current movement of the human being (100), the model data set corresponding to a data set which may be generated by a certain movement of the human being (100).

8. The device in accordance with claim 7, wherein the model data set comprises a parameter, and wherein the means (130) for evaluating is implemented to vary the parameter in order to maximize a percentage matching value.

9. The device in accordance with one of the preceding claims, wherein the means (130) for evaluating is implemented to store data of the means (110) for detecting a sound and/or data of the means (120) for acquiring an acceleration.

10. The device in accordance with one of the preceding claims, wherein the means (130) for evaluating comprises transfer means (132) and the transfer means (132) is implemented to acquire data from the means (110) for detecting a sound and/or data from the means (120) for acquiring an acceleration or transfer same to a central unit.

11. The device in accordance with claim 10, wherein the transfer means (132) acquires data in a wireless manner or transfers data in a wireless manner.

12. The device in accordance with one of the preceding claims, wherein the means (110) for detecting and the means (120) for acquiring and the means (130) for evaluating are housed together in a combined sensor (200).

13. The device in accordance with claim 12, wherein the combined sensor (200) comprises means for attaching to a thigh or leg (108) of the human being (100).

14. The device in accordance with one of the preceding claims, wherein the means (110) for detecting comprises means for attaching to a thigh (108) and the means (120) for acquiring an acceleration comprises means for attaching to a hip area (107) of the human being (100).

15. The device in accordance with one of the preceding claims, further comprising further means for acquiring cardiac muscle sounds, wherein the further means may be attached close to the heart (105) of the human being (100) and the means for evaluating (130) is implemented to perform determining the loss of control also from the cardiac muscle sounds.

16. The device in accordance with one of the preceding claims, wherein the means (110) for detecting and the means (120) for acquiring or the combined sensor (200) comprise means for attaching to a head-neck area (102) to determine head nodding resulting from over-tiredness of the human being (100).

17. The device in accordance with one of the preceding claims, wherein the means (110) for detecting and/or the means (120) for acquiring comprise wireless transmitting means, the wireless transmitting means being implemented to transmit sensor data to the means (130) for evaluating.

18. The device in accordance with one of the preceding claims, wherein the means (130) allows transmitting data by means of Bluetooth.

19. The device in accordance with one of the preceding claims, further comprising further means for detecting further sounds originating outside the human being (100), and wherein the means (130) for evaluating is implemented to receive data from the further means for detecting further sounds and to form a sound difference between the sounds and the further sounds.

20. The device in accordance with one of the preceding claims, wherein the means (120) for acquiring an acceleration is implemented to determine a timeline of the position of a torso (104) of the human being (100) relative to the gravitational field.

21. The device in accordance with one of the preceding claims, wherein the means (130) for evaluating is implemented to receive data of the means (110) for detecting a sound in predetermined time intervals, and is further implemented to additionally receive, when reaching a sound threshold value, data from the means (120) for acquiring an acceleration in order to generate a warning signal (135) when an acceleration threshold value is exceeded.

22. The device in accordance with claim 21 wherein the sound threshold value represents a bandwidth of a sound pattern, and wherein the acceleration threshold value comprises violation of a certain acceleration value.

23. The device in accordance with claims 21 or 22, wherein the means (130) for evaluating is implemented to acquire acceleration data from the means (120) for acquiring the acceleration within a predetermined time interval after the means (110) for detecting has acquired a sound above or below the sound threshold value and to generate the warning signal (135) when the acceleration threshold value is exceeded.

24. A method for predicting a loss of control over a muscle of a human being (100), comprising:
detecting a sound in a muscle;
acquiring an acceleration of the human being; and
evaluating the sound and the acceleration in order to determine an imminent loss of control over the muscular system from the sound and/or the acceleration.

25. The method in accordance with claim 24, wherein the step of detecting the sound takes place at a first position of the body and the step of acquiring the acceleration takes place at a second position of the body and the method additionally comprises a step of optimizing, the step of optimizing comprising altering the first and/or second positions of the body so that improved detection of the sound and improved acquiring of the acceleration are achieved.

26. The method in accordance with claim 25, wherein the step of optimizing is performed in such a way that sounds indicating a potential loss in tone are clearly detectable.

27. The method in accordance with one of claims 24 to 26, further comprising:
detecting environmental sounds; and
forming a difference signal,
wherein a source of the environmental sounds is outside the muscle and is of an origin deviating from that of the loss in tone, and the difference signal is formed from the sound of the muscle and the environmental sound so that sensitivity for muscle sounds resulting from an imminent loss in tone is increased.

28. A computer program comprising a program code for performing the method in accordance with claim 24 when the program runs on a device in accordance with claim 1.

## Revendications

1. Dispositif de prédiction d'une perte de contrôle sur un muscle d'un être humain (100), avec:
un moyen (110) destiné à détecter un bruit de muscle;
un moyen (120) destiné à capter une accélération de l'être humain (100); et
un moyen (130) destiné à évaluer le bruit et l'accélération, pour constater, à partir du bruit et de l'accélération, une perte imminente du contrôle sur le muscle.

2. Dispositif selon la revendication 1, dans lequel le moyen (110) destiné à détecter est réalisé pour détecter le bruit d'un muscle de support ou d'un muscle de la jambe et dans lequel le moyen (130) destiné à évaluer est réalisé pour constater une perte de contrôle imminente du muscle de support ou du muscle de la jambe.

3. Dispositif selon la revendication 1 ou la revendication 2, dans lequel le moyen (130) destiné à évaluer est réalisé pour sortir, en cas de constatation d'une perte imminente de contrôle de la musculature, un signal d'avertissement (135) pouvant être capté par l'être humain (100).

4. Dispositif selon la revendication 3, dans lequel le signal d'avertissement (135) comporte un signal lumineux et/ou un signal acoustique et/ou une stimulation notable.

5. Dispositif selon la revendication 3 ou la revendication 4, dans lequel le moyen (130) destiné à évaluer est réalisé pour transmettre le signal d'avertissement (135) à un endroit d'avertissement prédéterminé.

6. Dispositif selon l'une des revendications précédentes, dans lequel le moyen (130) destiné à évaluer est réalisé pour capter le bruit et l'accélération à des intervalles de temps et pour les combiner pour former un ensemble de données.

7. Dispositif selon la revendication 6, dans lequel le moyen (130) destiné à évaluer est réalisé pour comparer l'ensemble de données à un ensemble de données modèle et pour déterminer une valeur de coïncidence en pour cent, pour conclure d'un mouvement actuel de l'être humain (100), l'ensemble de données modèle correspondant à un ensemble de données pouvant être généré par un mouvement déterminé de l'être humain (100).

8. Dispositif selon la revendication 7, dans lequel l'ensemble de données modèle présente un paramètre et dans lequel le moyen (130) destiné à évaluer est réalisé pour faire varier le paramètre, pour maximiser une valeur de coïncidence en pour cent.

9. Dispositif selon l'une des revendications précédentes, dans lequel le moyen (130) destiné à évaluer est réalisé pour mémoriser des données du moyen (110) destiné à détecter un bruit et/ou des données du moyen (120) destiné à capter une accélération.

10. Dispositif selon l'une des revendications précédentes, dans lequel le moyen (130) destiné à évaluer présente un moyen de transmission (132) et le moyen de transmission (132) est réalisé pour capter ou pour transmettre à une unité centrale des données du moyen (110) destiné à détecter un bruit et/ou des données du moyen (120) destiné à capter une accélération.

11. Dispositif selon la revendication 10, dans lequel le moyen de transmission (132) capte sans fil ou transmet sans fil les données.

12. Dispositif selon l'une des revendications précédentes, dans lequel le moyen (110) destiné à détecter et le moyen (120) destiné à capter et le moyen (130) destiné à évaluer sont logés ensemble dans un capteur combiné (200).

13. Dispositif selon la revendication 12, dans lequel le capteur combiné (200) présente un moyen destiné au placement sur une cuisse ou une jambe (108) de l'être humain (100).

14. Dispositif selon l'une des revendications précédentes, dans lequel le moyen (110) destiné à détecter présente un moyen destiné au placement sur une cuisse (108) et le moyen (120) destiné à capter une accélération présente un moyen pour le placement sur une zone de hanche (107) de l'être humain (100).

15. Dispositif selon l'une des revendications précédentes, présentant par ailleurs un autre moyen destiné à capter des bruits du myocarde et l'autre moyen pouvant être placé près du coeur (105) de l'être humain (100) et le moyen destiné à évaluer (130) étant réalisé pour procéder à la constatation de la perte du contrôle également à partir des bruits du myocarde.

16. Dispositif selon l'une des revendications précédentes, dans lequel le moyen (110) destiné à détecter et le moyen (120) destiné à capter ou le capteur combiné (200) présentent un moyen pour le placement sur une région de cou-tête (102), pour constater une oscillation longitudinale de la tête par suite d'une fatigue excessive de l'être humain (100).

17. Dispositif selon l'une des revendications précédentes, dans lequel le moyen (110) destiné à détecter et/ou le moyen (120) destiné à capter présente un moyen d'émission sans fil, le moyen d'émission sans fil étant conçu pour transmettre des données de capteur au moyen (130) destiné à évaluer.

18. Dispositif selon l'une des revendications précédentes, dans lequel le moyen (130) permet une transmission de données par Bluetooth.

19. Dispositif selon l'une des revendications précédentes, présentant par ailleurs un autre moyen destiné à détecter d'autres bruits ayant leur origine en-dehors de l'être humain (100), et le moyen (130) destiné à évaluer étant réalisé pour recevoir des données de l'autre moyen destiné à détecter d'autres bruits et pour former une différence entre les bruits et les autres bruits.

20. Dispositif selon l'une des revendications précédentes, dans lequel le moyen (120) destiné à capter une accélération est conçu pour déterminer une évolution dans le temps d'une position d'un torse (104) de l'être humain (100) par rapport au champ de gravitation.

21. Dispositif selon l'une des revendications précédentes, dans lequel le moyen (130) destiné à évaluer est réalisé pour recevoir à des intervalles de temps prédéterminés des données du moyen (110) destiné à détecter un bruit et est par ailleurs réalisé pour obtenir, lorsqu'une valeur de bruit limite est atteinte, en outre des données du moyen (120) destiné à capter une accélération, pour générer à partir de ces derniers, lorsqu'une valeur d'accélération limite est excédée, un signal d'avertissement (135).

22. Dispositif selon la revendication 21, dans lequel la valeur de bruit limite représente une largeur de bande d'un modèle de bruit et dans lequel la valeur d'accélération limite comporte une violation d'une valeur d'accélération déterminée.

23. Dispositif selon la revendication 21 ou la revendication 22, dans lequel le moyen (130) destiné à évaluer est réalisé pour capter des données d'accélération du moyen (120) destiné à capter l'accélération dans un intervalle de temps prédéterminé après que le moyen (110) destiné à détecter ait détecté un bruit au-dessus ou au-dessous de la valeur de bruit limite et pour générer le signal d'avertissement (135) lorsque la valeur d'accélération limite est excédée.

24. Procédé de prédiction d'une perte d'un contrôle sur un muscle d'un être humain (100), avec:
détecter un bruit de muscle;
capter une accélération de l'être humain; et
évaluer le bruit et l'accélération, pour constater, à partir du bruit et de l'accélération, une perte imminente du contrôle sur la musculature.

25. Procédé selon la revendication 24, dans lequel l'étape de détection du bruit a lieu à un premier endroit du corps et l'étape de captation de l'accélération a lieu à un deuxième endroit du corps et le procédé présente par ailleurs une étape d'optimisation, l'étape d'optimisation comprenant un changement du premier et/ou du deuxième endroit du corps, de sorte que soient obtenues une meilleure détection du bruit et une meilleure captation de l'accélération.

26. Procédé selon la revendication 25, dans lequel l'étape d'optimisation est réalisée en ce sens que les bruits indiquant une possible perte de tonus soient clairement détectables.

27. Procédé selon l'une des revendications 24 à 26, présentant par ailleurs:
une détection de bruits environnementaux; et
une formation d'un signal de différence,
une source de bruits environnementaux se situant en-dehors du muscle et présentant une origine différente de la perte de tonus, et le signal de différence étant formé à partir du bruit du muscle et du bruit environnemental, de sorte qu'une sensibilité aux bruits de muscle soit augmentée par suite d'une perte de tonus imminente.

28. Programme d'ordinateur avec un code de programme pour réaliser le procédé selon la revendication 24 lorsque le programme est exécuté sur un dispositif selon la revendication 1.
